# EUROPEAN PATENT APPLICATION

(11) **EP 1 211 314 A2**
(43) Date of publication of application: **05.06.2002**
(21) Application number: 01121987.0
(22) Date of filing: 13.09.2001
(51) Int. Cl.: C12N 15/17, C12N 15/62, C12N 15/81, C12N 15/90, C12N 1/16, C07K 14/62

(54) **Expression of a human insulin precursor in p. pastoris**

(30) Priority: 13.09.2000 AR 0104797
(71) Applicant: LABORATORIOS BETA S.A., C1232AAR Buenos Aires (AR)
(72) Inventor: Annibali, Nestor, 1428 Buenos Aires (AR)
(74) Representative: Gervasi, Gemma, Dr.

(57) **Abstract**

Methylotrophic recombinant yeast strain producing human insulin precursor, the strain comprising, in its genome, a copy of a first DNA construction and a second DNA construction, wherein the constructions are capably of directing the expression and secretion of human insulin precursor of the formula B(1-30)-Y1-Y2-A(1-21), wherein. Y1 is lysine or arginine; Y2 is lysine or arginine; B(1-30) is the B peptide of the human insulin; and B(1-21) is the A peptide of human insulin, and wherein the strain is yeast *Pichia Pastoris.* DNA constructions and method for obtaining the strain are also provided.

## Description

### BACKGROUND OF THE INVENTION

### 1. Field of the Invention.

The present invention relates to the expression of human insulin in *P. pastoris* and, more particularly the invention is related to the field of DNA recombinant technology and to the production of insulin precursors in host microorganisms such as yeast. More precisely, the invention refers to recombinant methylotrophic yeast strain for producing human insulin precursors. The invention also relates to DNA constructions and method for obtaining the strains. The inventive strain comprises, in its genome, at least one copy of a first DNA construction and one copy of a second DNA construction, wherein said constructions are capable of conducing the expression and secretion of an insulin precursor.

### 2. Description of the Prior Art.

It is well known that the disease of Diabetes is usually treated with injections of insulin, e.g. human insulin. Insulin is a central hormone of the metabolism and is a protein consisting of two polypeptide chains, namely A chain and B chain. A chain comprises 21 amino acids residues and B chain comprises 30 amino acid residues, and both chains are covalently connected by di-sulphur bridges in the positions A7-B7 and A20-B19, and by an intra-catenary di-sulphur bridge connecting the residues A6-A11.

The insulin is produced in the pancreas by the β cells of the Langerhans islets as preproinsulin. The preproinsulin consists of a prepropeptide having 24 amino acids actuating as an exporting signal sequence followed by a peptide named proinsulin and containing 86 amino acid residues. Said preproinsulin may be represented by: prepetide-B-C-A, wherein the C peptide is a connector peptide comprising 31 amino acid residues and chains A and B are the chains A and B of the human proinsulin.

When the preproinsulin chain is synthesized, the signal peptide directs the synthesis into the endoplasmic reticulum of the β cells, in that moment the signal peptide is split secreting the proinsulin into the reticulum.

Then, during the packing of the insulin molecule within the secreting system of the β cells, the peptide C is split secreting the native insulin molecule, properly "folded". The split of peptide C is carried out under the action of enzymes actuating on the proinsulin dibasic sequences.

Presently it is known that the peptide C carries out an important function in the formation of the tertiary structure of the insulin molecule.

The production of insulin for treating Diabetes is a concern for the pharmaceutical industry since many years ago. As from the development of the recombinant DNA techniques, a wide variety of method for the production of insulin in microorganisms has been published in several media.

The first host microorganisms employed in the recombinant DNA techniques were the bacteria, particularly the *Escherichia coli (E.coli)*. In the first tests using *E. coli,* strategies similar to those used in the production of synthetic insulin have been employed. According to these methods, chains A and B were cloned and expressed in the host microorganisms in an independent manner, thus obtaining two polypeptide corresponding to chains A and B. The native insulin was then obtained by carrying out, in *vitro,* the steps of forming the di-sulphur bridges between chains A and B and the respective intra-catenary bridge. This oxidizing process were carried out as it is disclosed by Chance, R.E. et al., in Diabetes care 4:147; 1981; and Goedel, D.V. et al. in Proc. Natl. Acad. Sci. U.S.A. 76: 106-110, 1979. One of the most important drawbacks of these methods is the random formation of the di-sulphur bridges generating molecules with an incorrect tertiary structure. By this method, the yielding of native insulin with biological activity is extremely low, thus dramatically increasing the production costs.

Considering the above mentioned difficulties the idea has arose in the experts of cloning the DNA sequence corresponding to the proinsulin or its derivatives wherein the peptide C is represented by fragments having several sizes. These ides have been based in that the presence of the peptide C or its derivatives produced a higher yield of proinsulin correctly folded after the oxidizing step as compared to the yield resulting from the oxidizing of chains A and B by separate (Dteiner, D.F. et al. Proc. Acad. Sci. 60:622; 1968). Thus, it was observed that chain C operating like a connecting peptide of chains B and A allows the cysteine residues are spatially favored for a correct oxidation. It was demonstrated that the thus formed proinsulin molecule could function like a precursor from which insulin could be obtained by removing, *in vitro,* by means of specific enzymes, the peptide C. (Kemmler, W. et al. J- Biol. Chem. 91: 246:6786; 1971). It was also demonstrated that if the fragment C of these precursors was changed by a connecting peptide of a smaller size and maintaining at both ends sites to be split *in vitro* by the proper enzymes action, results equivalent, and in some cases better, to the insulin production, were obtained. These precursors were named mini-proinsulins (Wollmer, A. et al. Hoppe-Seyler's, Z. Physiol. Chem. 355:1471-1476; 1974 and EP Patent 195 691).

European Patent No. discloses a process for producing and expressing insulin in *E. coli* and method for producing human insulin. The production in large scale or commercial scale of proinsulin in E. coli is disclosed in US 5,460,954. US 4,431,740 discloses a DNA having a sequence encoding proinsulin, and another DNA encoding pre-proinsulin, and a microorganism such as *E*. *coli* transformed with such sequences.

However, the expression of heterologous proteins in *E. coli* has a number of difficulties well known by the experts in the art. Briefly, the following can be mentioned.

When an *E. coli* or any other pro-karyotic microorganism is used as a host for the expression of proteins from eukaryotes, the microorganism is incapable of establishing the di-sulphur bridges for permitting the correct formation of the tertiary structure. As a consequence, when proteins such as the human insulin are cloned and expressed in microorganisms, such proteins tend to aggregate forming inactive complexes or inclusion bodies.

The solubilization and purification of proinsulin from the inclusion bodies requires of a large number of additional steps. One of these steps comprises dissolving the aggregates with reagents such as Urea or Guanidine chloride. Subsequently, it is necessary subjecting the insulin precursor to an oxidizing agent by means of oxidative sulfitolysis, wherein the cysteine molecules of both chains adopt the SSO⁻₃ form. Subsequently, the groups S-sulfonated are converted into sulphydryl groups (-SH-) in the presence of thiolated agent (di-thioteitrol or mercaptoethanol). Finally, these groups are oxidized in presence of oxygen for forming the sulfide bridges.

The methods for recovering proinsulin from the inclusion bodies are still the aim of several investigations, attempting to improve the yield and the correct folding of the protein dramatically reduced by the purification of the protein and causes the purification process to be extremely complex. (Chance, R. et al. Proceeding of the Seventh American Peptide Chemistry Symposium, pages 721-728; 1981; Pierce Chemical Company, Rockford, IL.; Chan, S.J. et al. Proc. Natl. Acad. Sci. USA 78 (9): 5401-5405, 1981, and Frank, B.H. et al. Proceeding of the Seventh American Peptide Chemistry Symposium, pages 729-739; 1981; Pierce Chemical Company, Rockford, IL.).

In addition, with the *E. coli* or any other prokaryote organism the translation of the proteins are begun with methionine amino acid. For eliminating the methionine from the terminal amino end the gene of interest is usually cloned as a fusion protein. The separation of the insulin from the fusion peptide requires an additional step involving the digestion of the peptide with specific protease. Otherwise, the methionine residue must be eliminated by cyanogen bromide (CNBr).

The European Patent No. 0 055 945 discloses a method and a vector for splitting a proinsulin analogous having a peptide C that is smaller and wherein the methionine residue is eliminated by employing a treatment with CNBr.

Other difficulties and drawbacks that may be found in the expression of heterologous proteins in prokaryotes is the decreasing or diminishing of the protein stability under the action of the cytoplasmic protease. US Patent No. 5,460,954 discloses a process fro producing human proinsulin in *E. coli* comprising a vector containing a sequence in 5' end of the gene of proinsulin, encoding an amino acid sequence preventing the degradation by protease within the cell.

Many investigators are attempting to improve the methods of producing human insulin in *E. coli* for obtaining a simpler method with better results. These methods for improving the protein yielding are carried out by replacing the peptide C by smaller sequences (Chang, Seung-Gu at al. *Biochem. J*. 329; 631-635, 1998).

Methods for expressing proinsulin in bacteria have also been developed, these methods combining different procedures such as the expression of a fusion protein comprised of a polyhistidine tale in the N-terminal end, a methionine residue and the sequence of the proprotein of the human insulin, all incorporated in an expression vector for bacteria (Cowley, Darrin J. et al. *FEBS Letters,* 402: 124-130,1997).

On the basis of the operative drawbacks and difficulties found in the expression of human insulin in prokaryotic hosts, many attempts have been made to obtain high expressions of human insulin in eukaryiotic hosts such as yeast. Consequently, the yeast has become one of the selected hosts for the expression of eukaryotic proteins. These microorganisms provide clear advantages as compared to the bacteria in relation to the production of mammal proteins. The yeast has secretary mechanisms that are similar to the secretary system of the mammals and has the capacity of folding, of proteolitically processing, of glycosilating and secreting, in a proper manner, the mammal proteins.

When appropriate vectors are employed in the yeast for exporting the protein outside the cell, the process of recovering and purification of the proteins exported to the culture medium is simpler and has a better yielding relative to the expression in the cell cytoplasm. In addition, the secretion system provides an appropriate environment for the formation of the di-sulfide bridges that are necessary for the folding of the proteins (Smith, et al. 1985; *Science* 229:1219). On the other side, the cytoplasm is a reducing environment wherein these connections are not produced. Under these circumstances, the proteins that need forming di-sulfide bridges for maintaining a correct tertiary structure, as it is the case of the insulin, can be produced with better results when the same are secreted.

Among the systems employing the yeast for operating as hosts for the production of a large number of proteins, the yeast of the species *Saccharomyces cerevisiae* may be found. The genetics of this yeast has been studied in detail by a number of investigation groups.

Several polypeptides such as the insulin have been cloned and expressed in *Saccharomyces cerevisiae.* The expression of this propeptide may follow the secretory way or may be accumulated in the cytoplasm of the host microorganism. In the event of the accumulation, time consuming and complex processes for purification must be employed, the processes requiring steps for the formation of the di-sulfide bridges, as it is disclosed in the European patent No. 37255. For avoiding these drawbacks and complicate steps, the sequence corresponding to the gene of proinsulin is cloned subsequently to an additional DNA sequence named "leader" or signal peptide that originates the pre-proinsulin peptide. This peptide, once recognized and processed by the yeast, provides the secretion of the proinsulin into the culture medium.

In addition to the foregoing, the precursor of the insulin type that are produced in *Saccharomyces cerevisiae* suffer from a rapid enzymatic process either when they are expressed in the cytoplasm as well as when they are secreted into the medium. It has been demonstrated that the human proinsulin is specially sensitive to enzymatic cuts in two dibasic sequences (Arg₃₁ - Arg₃₂ and Lys₆₄ - Arg₆₅). This causes the split of the molecule before the formation of the di-sulfide bridges, resulting in the generation of the peptides C, A and B separately.

It has been found that if, instead of proinsulin, shorter sequences are employed wherein the peptide C is removed or, it is simply represented by shorter fragments having up to two amino acids of the type of lysine, arginine, a molecule is obtained that is more stable, not digestible by proteases, and capable of been processed *in vitro* finally originating a biologically active insulin molecule (Lars Thim et al. Proc. Natl. Acad. Sci. USA 83: 6766-67770; 1986).

European Patent No. 195 691 discloses several precursors such as of the type B-X-Y-A wherein B and A corresponds to chains B and A of the human insulin, and wherein X and Y are represented by the amino acids lysine and arginine, these amino acids being digestible by the enzymes trypsine and carboxypeptidase B for its conversion to human insulin. However, while considerable amounts of A₀Arg-desB(30) are produced as sub-products of the digestion, this sub-product does not have the amino acid 30 of B chain and an arginine residue remains connected to A chain. The arginine residue can not be easily removed thus causing serious inconveniencies in the process of purifying the protein, also considerably diminishing the production yields. The total production of this precursor en *Saccharomyces cerevisiae* is remarkably low.

On the other hand, US Patent No. 4,916,212 discloses a simple-chain proinsulin precursor, wherein said precursor is represented by the formula: B₍₁₋₂₉₎-(Xₙ-Y)ₘ-A₍₁₋ ₂₁₎, wherein Xₙ is a peptidic chain win n amino acids, Y is lysine or arginine, n is an integer from 0 to 35, m is 0 or 1, B₍₁₋₂₉₎ is a B chain lacking the threonine at position, and A₍₁₋₂₁₎ is A chain of the human insulin. This US Patent discloses -Xₙ-Y- as not containing two adjacent basic amino acids, such as lysine and arginine, because the digestion with trypsin produces sub products that are difficult to separate during the purification steps. The products obtained from these genetic designs do not contain the aminoacid threonine at position 30 and, therefore, they must be subjected to an additional step consisting of the addition of this amino acid by means of the catalytic action of the trypsin in presence of the Thr-Obu ester, as it is disclosed in the US Patent No. 4,343,898 and Rose, K. et al. *Biochem. J*. 211:671-676, 1983.

In any case, in addition to all the modifications carried out in the insulin precursors, the expression of these peptides in *Saccharomyces cerevisiae* has resulted in low yieldings and drawbacks related to the scale of production of heterologous protein. These problems are generally associated to low efficiency promoters and to the fact that the sequences of interest are cloned in autonomous replication plasmides. These plasmides are not kept uniformly distributed in the culture and they usually diminish in the number of copies. As a result of this, and after some duplication cycles, cells with 2, 3 or 0 copies of the plasmide used as a vector are found in the culture (Chan, S.J. et al. *Proc. Natl. Acad. Sci.* USA 78 (9): 5401-5405. 1981).

An expression system in yeast, that is distinct from the one using *Saccharomyces* as a host, is the system of the methylotrophic yeast. These microorganisms may be very useful as hosts for the expression of heterologous proteins, which proteins are required in large production volumes. The heterologous proteins that are expressed in methylotrophic yeast may be secreted with expression levels that are equivalent to the ones of *E.coli* and that are higher as compared to the expression levels of the *Saccharomyces cerevisiae.*

The methylortrophic yeasts are unicellular microorganisms capable of growing in presence of methanol as the only one carbon source. This yeast can be kept without incoveniencies in high cellular densities when they are cultured in high volume fermentor. In addition, this yeast is capable of producing many of the pos-translated modifications carried out by the upper eukaryotic cells, such as proteolytic digestions, protein folding, di-sulfide bridges formation and glycosilation.

*Pichia pastoris* is one of the twelve species within the four yeast genus capable of metabolizing methanol as the only one carbon source (Cregg, J.M. et al. *Bio/Technology* 11:905-910, 1993). The remaining genus are represented by *Candida, Hansenula* and *Torulopsis.*

This yeast comprise a large number of enzymes corresponding to the metabolic pathways of methanol (Veenhuis, M. et al. *Adv. Microb. Physiol.* 24:1-82, 1983). The first step of this metabolic pathway is the oxidizing of methanol to formaldehyde, generating hydrogen peroxide under the action of the alcohol oxidase enzyme (AOX).

The cell prevents the hydrogen peroxide from toxicity by carrying out this first metabolism reaction of the methanol in special organelle named peroxisome.

There are two genes in *P. pastoris* encoding alcohol oxidase enzymes I and II, AOX1 and AOX2 genes. The AOX2 gene is responsible of the most part of the alcohol oxidase activity in the cell (Cregg, J.M. et al. *Mol. Cell. Biol.* 9:1316-1323, 1989).

The expression of this gene is highly regulated and it is induced by the methanol, with the AOX1 representing a value close to the 30% of the total soluble proteins of the cell. Because of this the expression systems most employed in *Pichia pastoris* include in their vectors the AOX1 gene promoter.

Thomas Kjeldsen et al. carried out a comparison between the expression of proinsulin and precursor peptides of (B₁₋₂₉ - Ala-Ala-Lys - A₁₋₂₁) insulin in *S*. *cereviciae* and in *P*. *pastoris.* The products were secreted in the culture medium through an amino acid sequence that is fused to the amino termination end of the precursor termed leader. For determining the secretion efficiency of the insulin precursor several signal peptides were employed such as pre-pro-peptide α mating factor of *Saccharomyces cerevisiae* and synthetic derivatives thereof. These pre pro peptides have amino acid sequences useful for target for the action from specific proteases permitting the liberation of the peptide into the culture medium. All the insulin peptides employed by these authors are secreted into the medium as a precursor lacking threonine at position 30 of B chain. This product, recovered and purified from the culture medium, had to be subject to an exhaustive process called transpeptidation. The transpeptidation consists of the addition of threonine and it is disclosed in US Patent No. 4,916,212 to Markussen et al. The transpeptidation includes an additional step in the purification process of the insulin molecule.

In the above described state of the art, it has been a concern of the inventors to find a solution to all of the above mentioned problems and drawbacks in the art.

### SUMMARY OF THE INVENTION

It is therefore one object of the present invention to provide a new yeast strain capable of producing and secreting to the medium an insulin precursor in proper quantities useful for its industrial application, wherein the inventive strain comprises two distinct DNA constructions for expressing a DNA sequence encoding an insulin precursor. Said gene is cloned in such a way that an insulin precursor is secreted into the medium, the insulin precursor containing at its termination end the first amino acid corresponding to the insulin B-chain, thus avoiding the steps for eliminating the remaining aminoacids from the signal peptide.

It is a further object of the present invention to provide a methylotrophic recombinant yeast strain for producing human insulin precursor, the strain having a genome comprising a copy of a first DNA construction and a second DNA construction, wherein said constructions controlling the expression and secretion of a human insulin precursor, said DNA constructions comprising at least one DNA sequence encoding a human insulin precursor or analogues thereof.

It is even another object of the present invention to provide a yeast strain comprising in its genome DNA constructions capable of expressing a human insulin precursor of the formula:

B(1-30)-Y1-Y2-A(1-21), wherein Y1 is lysine or arginine; Y2 is lysine or arginine; B(1-30) is the B peptide of the human insulin; and B(1-21) is the A peptide of human insulin.

It is even another object of the present invention to provide a *Pichia pastoris* strain deposited on July 25, 2000, in the American Type Culture Collection (ATCC) under number PTA-2260, wherein the yeast strain comprises, in its geneome, a first DNA construction comprising:
a) a first insertable DNA sequence corresponding to a 5' regulatory region of *Pichia pastoris* AOX1 gene, operably linked to
b) the MF α signal sequence of *Sacharomyces cerevisiae,* operable linked to
c) the sequence encoding a human insulin precursor, preferably a precursor of formula B(1-30)-Y1-Y2-A(1-21) operable linked to
d) a 3' termination sequence of *Pichia pastoris* AOX1 gene operably linked to
e) a *Pichia pastoris* HIS4 selection gene operably linked to
f) a second insertion sequence corresponding to *Pichia pastoris* AOX1 gene 3' end; and
a second DNA construction comprising:
a) a first insertable DNA sequence corresponding to a 5' regulatory region of *Pichia pastoris* AOX1 gene operably linked to
b) the MF α signal sequence of *Sacharomyces cerevisiae* operable linked to
c) the sequence encoding a human insulin precursor, preferably a precursor of formula B(1-30)-Y1-Y2-A(1-21). operable linked to
d) a 3' termination sequence of *Pichia pastoris* AOX1 gene operably linked to
e) the zeocine-resistant selection gene.

It is still another object of the present invention to provide a first DNA construction comprising at least one expression cassette for expressing the human insulin precursor, the cassette comprising:
a) a 5' regulatory region operably linked to
b) a DNA sequence encoding a signal sequence operable linked to
c) a sequence encoding a human insulin precursor operable linked to
d) a functional termination sequence.

According to an embodiment of the invention, the first DNA construction comprises, at its 5' and 3' ends, sequences homologous with a target gene of the yeast enough to permit the insertion by gene replacement of the DNA construction in the target gene, in the same relative orientation of the target gene in the yeast genome, these 5' and 3' sequences that are homologous to the target gene being sequences flanking the expression cassette.

It is even another object of the present invention to provide a
first DNA construction of claim 5, further comprising:
a) a first insertable DNA sequence corresponding to a 5' regulatory region of *Pichia pastoris* AOX1 gene operably linked to
b) the MF α signal sequence of *Sacharomyces cerevisiae* operable linked to
c) the sequence encoding a human insulin precursor, preferably a precursor of formula B(1-30)-Y1-Y2-A(1-21) operable linked to
d) a 3' termination sequence of *Pichia pastoris* AOX1 gene operably linked to
e) a *Pichia pastoris* HIS4 selection gene operably linked to
f) a second insertion sequence corresponding to 3' termination sequence of *Pichia pastoris* AOX1 gene.

It is a further object of the present invention to provide a second DNA construction comprising at least one expression cassette for expressing the human insulin precursor, the cassette comprising:
a) a 5' regulatory region operably linked to
b) a DNA sequence encoding a signal sequence operable linked to
c) a sequence encoding a human insulin precursor operable linked to
d) a functional termination sequence.

According to a preferred embodiment of the invention, the second DNA construction comprises a selection marker gene distinct from the selection marker gene of the first DNA construction, thus permitting a second selection of the inventive transformed yeast strain.

According to another embodiment of the invention, the second DNA construction comprises a single sequence homologous enough with a target gene of the yeast, allowing the integration of the DNA construction in the target gene, in a single event.

It is even another object of the present invention to provide a second DNA construction comprising:
a) a first insertable DNA sequence corresponding to a 5' regulatory region of *Pichia pastoris* AOX1 gene operably linked to
b) the MF α signal sequence of *Sacharomyces cerevisiae* operable linked to
c) the sequence encoding a human insulin precursor, preferably a precursor of formula B(1-30)-Y1-Y2-A(1-21) linked to
d) a 3' transcription termination sequence of *Pichia pastoris* AOX1 gene linked to
e) the zeocine-resistant selection gene.

According to an embodiment of the invention, in both DNA constructions, the sequence encoding the human insulin precursor is cloned in said construction following the site of protease, wherein all secreted human insulin precursor contains, in its amino terminal, the fenilalanine amino acid.

Also according to an embodiment of the invention, each of the DNA constructions is incorporated into a vector selected from the group consisting of linear and circular vectors.

It is even another object of the present invention to provide a method of obtaining a transformed methylotrophic yeast strain for producing high quantities of a human insulin precursor, the method comprising the steps of:
i) transforming a yeast cell with a first DNA construction comprising:
   a) a first insertable DNA sequence corresponding to a 5' regulatory region of *Pichia pastoris* AOX1 gene operably linked to
   b) the MF α signal sequence of *Sacharomyces cerevisiae* operable linked to
   c) the sequence encoding a human insulin precursor, preferably a precursor of formula B(1-30)-Y1-Y2-A(1-21) operable linked to
   d) a 3' transcription termination sequence of *Pichia pastoris* AOX1 gene operably linked to
   e) a *Pichia pastoris* HIS4 selection gene operably linked to
   f) a second insertion sequence corresponding to 3' end of *Pichia pastoris* AOX1 gene;
ii) selecting the yeast cells;
iii) isolating a yeast strain;
iv) re-transforming the yeast strain obtained in steps i)-iii) with a second DNA construction comprising:
   a) a first insertable DNA sequence corresponding to a 5' regulatory region of *Pichia pastoris* AOX1 gene operably linked to
   b) the MF α signal sequence of *Sacharomyces cerevisiae* operable linked to
   c) a sequence encoding a human insulin precursor, preferably a precursor of formula B(1-30)-Y1-Y2-A(1-21) linked to
   d) a 3' transcription termination sequence of *Pichia pastoris* AOX1 gene linked to
   e) the zeocine-resistant selection gene;
v) selecting the re-transformed yeast strain; and
vi) isolating the selected and re-transformed yeast strain.

It is still another object of the present invention to provide an insulin precursor secreted into the medium as a precursor containing threonine at position 30 of B chain, thus avoiding the complex and cumbersome transpeptidation step.

The above and other objects, features and advantages of this invention will be better understood when taken in connection with the accompanying drawings and description.

### BRIEF DESCRIPTION OF THE DRAWINGS

The present invention is illustrated by way of example in the following drawings wherein:
FIG. 1 provides a restriction map of plasmid pPIC9-Ib; and
FIG. 2 provides a restriction map of plasmid pPICZαA-Ib;

### DESCRIPTION OF THE PREFERRED EMBODIMENTS

Unless defined in another way the technical and scientific terminology utilized in the present description has the meaning as commonly interpreted by the person skilled in the art of the invention. All the patents and publications mentioned in the present application are incorporated herein as a reference only.

### Definitions

The term "human insulin precursor" or "proinsulin" as used herein refers to any human insulin precursor or analogue thereof originating an insulin molecule or related molecules showing the same biological activity of the insulin.

The meaning of "biological activity" is the biological activity associated to insulin evaluated through tests known for the person skilled in the art.

As used herein, the insulin precursors include the allelic variations of the insulin precursors and derivatives obtained through simple modifications of the amino acid sequence of the insulin product.

As used herein, the terms "leader sequence" or "signal sequence" are indistinct expressions and refer to amino acid sequences carrying out the transport of a peptide linked thereto through the cellular membrane.

As used herein, the term "a DNA construction" encircle an expression cassette and also other DNA sequences.

In order to resolve the above mentioned drawbacks and problems, the inventors have developed a new and inventive yeast strain expressing high quantities of a human proinsulin molecule. This yeast strain has been obtained by a new and inventive process or method comprising the steps of sequentially transforming and re-transforming the yeast with two distinct and inventive DNA constructions. The proinsulin secreted into the medium by the new strain is an insulin precursor, said precursor being preferably a precursor with its peptide C being replaced by a sequence of two amino acids and wherein the purification of same, for obtaining the active human insulin, generates few contaminants, thus avoiding the Trypsin-mediated transpeptidation steps without diminishing the required industrial yields or production. Also, said DNA constructions have been cloned in such a way to avoid the step of eliminating the remaining amino acids of the signal peptide of the secreted proinsulin.

By using the strain and the DNA construction of the present invention production levels of human insulin between 200 to 400 mg/liter of fermentation are obtained, such levels being considered very appropriate for industrial production.

The chain coding the human insulin precursor gene has been obtained through synthesis, by employing the polymerase chain reaction.

The gene synthesis methodology has the advantage of being rapid and permitting the election of the codons most utilized by the selected expression host.

This process comprises the chemical synthesis of an oligonucleotide group composing the entire sequence of both DNA chains of the selected precursors. Subsequently, the linking of complementary oligonucleotide pairs is carried out. For preventing the problems associated to cross hybridization events between the oligonucleotides, a PCR method has been employed, this method permitting to complete the process in only one day.

The first step consists of the production of a central template. Oligonucleotides located in the center of the sequence to be construed, the oligonucleotides being complementary to each other in their 3' ends, having an specific *Tm* for each pair of nucleotides.

Subsequently, a complete double chain has been obtained by PCR from the 3' end.

An aliquot of the PCR mixture has been employed in a second PCR event after the addition of the corresponding primers.

Subsequently, the process was continued with appropriate pairs of primers until obtaining the final product.

Once the DNA fragments encoding the human insulin precursor have been obtained, the fragments were incorporated into vector pPIC9 (Invitro). Once both entities were linked, the recombinant vector was characterized with restriction enzymes. Some of the randomly taken recombinant vectors were sequenced according to Sanger method employing the Kit Sequenace V 2.0. The primers employed for the sequencing of the strand 5'-3' were 5' AOX1 and factor α, and for the strand 3'-5' the primer 3' AOX1 was employed, both primer being provided by Invitrogen. The results of the sequence confirmed that, in the vector, the sequence of the proinsulin was correct.

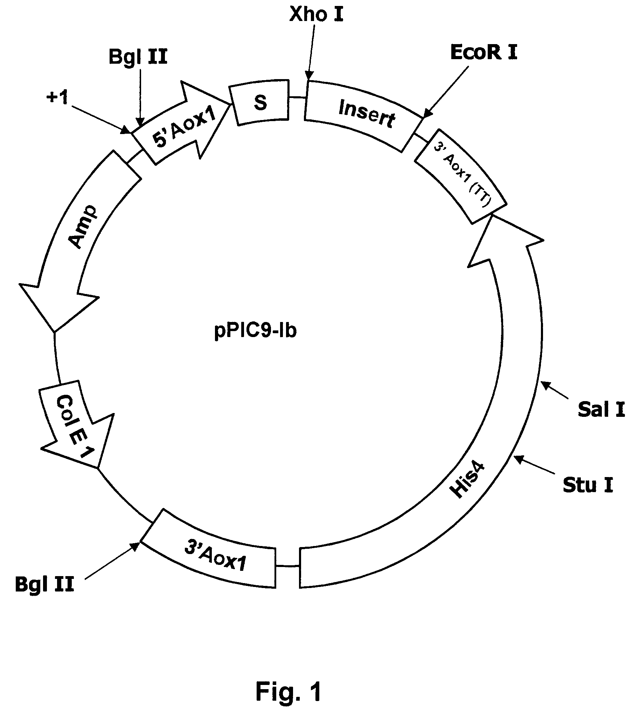

The new formed vector was termed pPIC9-Ib (Fig.1). The vector digested with the appropriate restriction enzyme originated two DNA fragments; the fragment containing the inventive DNA construction was employed for transforming the methylotrophic yeast. Said DNA construction comprises a methanol responsive sequence represented by the AOX1 gene promoter element of a methylotrophic yeast, a DNA sequence encoding a signal sequence, a human insulin precursor gene, the transcription termination signal sequence of the AOX1 gene and the HIS4 gene encoding histidinol dehydrogenase, all contained between 5' and 3' ends of the AOX1 gene.

According to the invention, as it is well known for any person skilled in the art, any circular or linear integrative site-specific vector may be utilized for the transformation of the yeasts.

In the DNA construction according to the invention, also any signal sequence permitting the proper exportation of the insulin precursor may be utilized. Preferably, the signal sequence MF α of *S. cerevisiae* that is a peptide comprised of 13 amino acid residues may be employed. The MF α signal sequence has a protease site determined by the sequence of amino acids Lys-Arg-Glu-Ala. During the cloning process in pPIC9 of a human insulin precursor gene, this gene may be preferably inserted -into the site Xho I that eliminates the -Glu-Ala- residues, whereby the starting insert of the human insulin gene is maintained immediately after the proteases removal site (Figure 1). The cloning in site Xho I permits to obtain a precursor released into the culture medium without remaining amino acids belonging to the signal peptide, thus simplifying the steps of purification of the human insulin.

There are several genes in the yeast which are included in the methanol metabolism pathway. The expression of these genes is controlled by their regulatory 5' regions responsive to methanol and known as promoters. Any of such regulatory 5' regions are proper for utilization as promoters in the DNA construction according to the invention. Examples of regulatory regions include, but are not limited thereto, the *Pichia pastoris* primary alcohol oxidase enzyme (AOX1) gene promoter, the secondary alcohol oxidase II enzyme (AOX2) gene promoter, the *P*. *pastoris* dihydroxyacetone synthase (DAS) gene promoter, the P. *pastoris* p40 gene promoter, the *P*. *pastoris* catalase gene promoter, and the glyceraldehide P dehydrogenase GAP promoter. Preferably, the *Pichia pastoris* primary alcohol oxidase enzyme (AOX1) gene promoter may be employed because this is highly efficient to provide high levels of expression. It will be apparent for any person skilled in the art that any of the promoters or regulatory regions selected are within the scope of the invention, the 5' regions being, however, the preferred ones because of their capacity of being responsive to an alcohol containing medium.

The 3' termination sequences of the DNA construction according to the invention are proper for terminating, polyadenylation and stabilizing the RNAm encoded by the insulin precursor gene. termination sequences that are characteristic of methylotrophic yeast families may be employed, preferably *Pichia pastoris* 3' termination sequences.

The DNA construction also contains a selectable marker gene. For these purposes any selection marker gene may be employed provided that the gene is functional in methylotrophic yeast including, but not limited thereto, any gene capable of providing a selected phenotype to the methylotrophic yeast, permitting positively selecting the yeast transformed with the DNA construction of the invention. An appropriate marker is any system utilizing a mutant auxotrophic *Pichia pastoris* host cell and the wild biosynthetic type gene complementing the host defects. Preferably, the HIS4 gene encoding the histidinol dehydrogenases and the auxotrophic mutant cell may be employed.

The DNA construction of the invention employed for transforming methylotrophic yeasts has the characteristic of being insertable into the genome of the host yeast through homologous recombination with 5' and 3' ends of the endogenous AOX1 gene of the yeast, wherein said endogenous gene is replaced by the DNA construction of the invention.

The DNA construction according to the invention may be inserted in any functional vector in bacteria (chimeric vector), wherein the vectors include selection markers and replication sites proper for the bacteria. This vector may have a circular shape forming extra-chromosomal replication plasmids within the bacteria. Several copies of the inventive DNA construction could be incorporated into said vector.

The inventive DNA construction is employed for transforming methylotrophic yeasts according to any standard method of transforming yeasts. Examples of transforming methods include, while they are not limited to the following, electroporation, spheroplasts, transformation with lithium chloride and transformation with PEG 1000; preferably, the method of spheroplasts and electroporation are employed. The transformation with the DNA construction can be carried out with the DNA construction arranged in linear o circular pattern. The DNA construction is directed to the target gene of the yeast genome by flanking sequences having enough homology with the target gene in order that the DNA construction is integrated to the site to which it was directed. In a different embodiment of the invention at least one copy of the DNA construction according to the invention is integrated to the host genome in the correct orientation.

It is also possible to employ any other methylotrophic yeast strain. Examples of methylotrophic strains include, while not limited thereto, the genes *Pichia, Torulopsis, Hansenula* and *Candida,* with the *Pichia pastoris* GS 115 strain (ATCC N° 20864) being preferably employed, this strain having the mutated HIS4 gene, therefore, it is HIS⁻.

With all the His⁺ transformers the inventive DNA construction integrated by replacing the structural AOX1 gene of the GS115 strain genome occurs with a frequency of about 5% to 35%. The replacement event of the structural AOX1 gene of the yeast genome generates yeasts called Mut^{s}, which are sensitive to the use of methanol as carbon source. Any expert in the art can understand that the inventive DNA construction can also be integrated by some of its 5' or 3' ends within the AOX1 gene generating Mut^{r} yeasts which are resistant to the use of methanol as carbon source because they keep the functional AOX1 gene; the DNA construction could also be integrated with the genome of the yeast by recombination with the His gene of the yeasts the sequence of which is also present in the DNA construction of the invention, or the DNA construction could be integrated in several sites of the yeast genome without restricting the scope of the invention.

Subsequently, the clones transformed with the inventive DNA construction are selected by any method known in the art but preferably replica plating experiments are carried out in permitting to distinguish His⁺ Mut^{r} clones and His⁺ Mut^{s} clones. Alternatively, clones with the capability of production can be selected by employing electrophoretic geles and immunochemical techniques.

Each of Mut^{s} / Mut^{r} clones selected by the above mentioned methods was sub-cloned and isolated as pure clones. Among all the selected clones those producing proper quantities of the insulin precursor were selected. These elected clones were characterized and the number of copies of the inventive DNA construction was analyzed. Several clones producing proper quantities of the insulin precursor were detected, with some of them being Mut^{s} and other ones being Mut^{r}.

Some of these clones were employed for subjecting the same to the second transformation event herein called also as re-transformation.

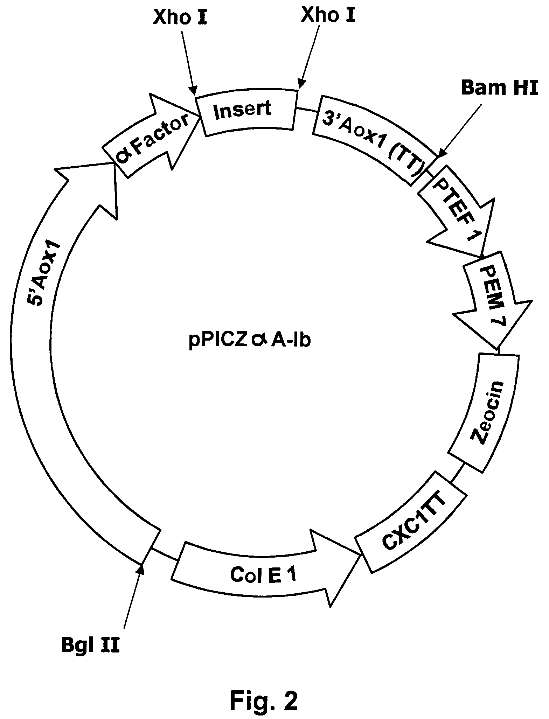

In another embodiment of the invention, the sequence of nucleotides encoding an insulin precursor was amplified by PCR, isolated and cloned in the pPICZαA vector in the specially designed multi-cloned site and a vector called pPICZαA (Figure 2) was obtained. Said vector contains a DNA construction, called second DNA construction, comprising a promoter responsive to methanol of methylotrophic yeast AOX1 gene; a DNA sequence encoding signal sequence, the insulin precursor gene, the signal sequence of the termination of the transcription and a selection gene distinct from the ones employed in the first DNA construction of the invention.

Any signal sequence appropriately permitting the sequencing of insulin precursor may be employed. Examples of signal sequences include, although they are not limited to the following, the MFα signal sequences of *s. Cerevisiae* and the signal sequence of alkaline phosphatase, with the MFα signal sequence of *s*. *cerevisiae,* corresponding to a peptide having 13 amino acid residues, being preferably employed. During the cloning process of the gene encoding the insulin precursor in the pPICZαA plasmid, said gene was inserted in the Xho I site that eliminates the -Glu-Ala-residues, whereby the beginning of the insulin precursor gene remained immediately after the proteases removal site. This cloning design permits the precursor released into the culture medium to be free of remaining amino acids of the signal peptide; thus avoiding one step in the purification sequence of the human insulin.

Any 5'regulatory sequence is proper for employing as a promoter in the second DNA construction of the invention. Examples of regulatory regions include, while they are not limited thereto, the *Pichia pastoris* primary alcohol oxidase enzyme (AOX1) gene promoter, the secondary alcohol oxidase II enzyme (AOX2) gene promoter, the *P.* *pastoris* dihydroxyacetone synthase (DAS) gene promoter, the *P. pastoris* p40 gene promoter, the *P. pastoris* catalase gene promoter, and the glyceraldehide P dehydrogenase GAP promoter. Preferably, the *Pichia pastoris* primary alcohol oxidase enzyme (AOX1) gene promoter may be employed because this is highly efficient to provide high levels of expression. It will be apparent for any person skilled in the art that any of the promoters or regulatory regions selected are within the scope of the invention. Preferably, the 5' regulatory regions that are capable of being responsive to an alcohol containing medium are employed.

The 3' termination sequences of the second DNA construction according to the invention are proper for terminating, polyadenylation and stabilizing the RNAm encoded by the insulin precursor gene. termination sequences that are characteristic of methylotrophic yeast families may be employed, preferably *Pichia pastoris* 3' termination sequences are employed.

The second DNA construction of the invention also contains a selectable marker gene. For these purposes any selection marker gene functional in methylotrophic yeasts may be employed, but different from the one employed in the prior transformation. It is preferred to employ the zeocine gene encoding for resistance to the zeocine antibiotics.

The new vector for the re-transformation of the yeasts may comprise a single copy or multiple copies of the second DNA construction of the invention. Any method for obtaining a vector with multiple copies may be employed, but preferably a method comprising a strategy of cloning multimerics is employed, generating a vector with multiple copies of the second DNA construction of the invention, preferably containing between 2 and 18 copies of said DNA construction.

The new isolated recombinant vectors are characterized by means of analysis with restriction enzymes. The recombinant vectors were sequenced and the correct position of the sequence encoding the insulin precursor and the signal peptide has been confirmed; and the number of copies of the gene of interest has also been determined.

The new vectors have been employed for the re-transformation of the yeasts. Vectors containing between 1 and 18 copies of the insulin precursor gene may be employed. Preferably, a vector containing a single copy of the second DNA construction of the invention may be utilized. Said recombinant vector may be linearized by digestion with restriction enzyme or may be utilized in the circular form for re-transforming the yeasts cells. Preferably, the linearized vector is utilized for the carrying out of the second transformation event in the Mut^{s} clones obtained in the prior transformation step.

Any method known in the art for the transformation of yeasts may be utilized in the re-transformation, these methods including, although they are not restricted to the following, spheroplasts, electropoarion, transformation with PEG 1000 and transformation with lithium chloride. Preferably, the spheroplasts transformation method or the electroporation are utilized.

Any expert in the art can understand that, for the carrying out of the second transformation event, vector with a single or multiple copies of the gene of interest may be employed, and wherein said transformation step can be carried out by employing any method of transforming yeasts without restricting or modifying the scope of the present invention.

The vector linearized with the inventive DNA construction utilized for re-transforming the methylotrophic yeasts clones producing the insulin precursor may preferably have the feature of being inserted into the host genome in only one site, subsequently generating multiple genomic copies *in vivo.*

The re-transformed clones have been properly selected by employing any of the known methods in order to carry out double selection of yeasts, preferably, employing a double selection in a medium without histidine and with zeocine.

The selected positive clones were isolated and purified, and the sequences integrated to the yeasts genome were characterized. The presence, in the total DNA, of re-transformed yeasts clones of the inventive DNA constructions were determined employing the Southern blot method and by means of a genomic analysis by PCR. The number of copies of the DNA construction of the present invention in the yeasts genome has been determined by employing the known Dot Blot method and by means of the method of analyzing the number of copies by PCR.

Among all the characterized clones the B1, 3.3 clone deposited on July 25, 2000 in the American Type Culture Collection (ATCC) under deposit number PTA-2260, was preferably selected, the clone containing a copy of the first DNA construction of the invention and-13 copies of the second DNA construction of the invention, wherein said clone is Mut^{s}, it is resistant to the zeocine and it can grow in histidine free medium.

Other production clones with the following features were isolated: C1, 46 clone: phenotype Mut^{r}, wherein the integration of the first DNA construction of the invention was in the His yeast gene, containing 5 copies of the DNA construction, and wherein said clone was subject to a single transformation event; C2, 7 clone: phenotype Mut^{s}, containing a single copy of the DNA construction and wherein said clone was subject to a single transformation event; clone 25; phenotype Mut^{r}, containing 6 copies of the DNA construction, the integration of the construction was in the yeast AOX 1 gene, and wherein said clone was subject to a single transformation event; clone V8, 10.1: phenotype Mut^{s}, with 8 copies of the second DNA construction of the invention generated *in vitro,* and wherein said clone appears after a re-transformation event of a clone containing at least one copy of the first DNA construction.

All the transformed and retransformed strains selected by their phenotypic and genotypic desired characteristic were cultured in Erlenmeyer flasks. The colonies and strain resulting of interest were selected to be cultured in fermenting devices.

For a large scale production of the insulin precursors, the typical method and processes used for methylotrophic yeasts were utilized; preferably, the fermentations were carried out by culturing the yeasts strains in the first step in a medium having a non inducing carbon source in excess, like glycerol. In this step, the expression of the inventive constructions with the gene encoding the human insulin precursor is totally repressed, generating an important biomass without insulin precursor expression. Subsequently to this growing period the cells were grown preferably under methanol restricting conditions with or without another carbon source for inducing the expression of the desired gene contained in the DNA constructions of the invention. Said DNA constructions were capable of expressing the gene encoding a human insulin precursor in reply to the methanol and were also capable of releasing or secreting significant quantities of the precursor into the culturing medium, in quantities enough and appropriate to be employed in the industrial scale.

The present invention will now be described with reference to certain examples which further illustrate but do not limit the invention.

### EXAMPLES

### Example 1

### Construction of the insulin precursor

The construction of an insulin precursor has been carried out by means of the Polymerase chain reaction (PCR), employing human codons:

The PCR conditions have been established according to the details of the publication A Method for Synthesizing Genes and cDNAs by Polymerase Chain Reaction. Di Donato, Alberto et al. Analytical Biochemistry. 212:291-293; 1993, modifying the annealing temperature according to the Tm of each oligonucleotide.

Primers:
SEQ ID: N°1: 5´-TCACACCTGG TGGAAGCTCT CTACCTAGTG
TGCGGG -3´
SEQ ID: N°2: 5´-GGTCTTGGGT GTGTAGAAGA AGCCTCGTTC
CCCGCACACT AGGTA-3´
SEQ ID: N° 3: 5´- TTTGTGAACC AACACCTGTG CGGCTCACAC
CTGGTGGAA -3´
SEQ ID: N°4: 5´-GCTGGTACAG CATTGTTCCA CAATGCCACG
CTTGGTCTTG GGTGT -3´
SEQ ID: N° 5: 5´-CTAGTTGCAG TAGTTCTCCA GCTGGTAGAG
GGAGCAGATG CTGGTACAGC AT-3´

Final Product:

SEQ ID: N° 6: 5´- TTTGTGAACC AACACCTGTG CGGCTCACAC

CTGGTGGAAG CTCTCTACCT AGTGTGCGGG GAACGAGGCT TCTTCTACAC

ACCCAAGACC AAGCGTGGCA TTGTGGAACA ATGCTGTACC AGCATCTGCT

CCCTCTACCA GCTGGAGAAC TACTGCAACT AG -3´

(complete insulin precursor)

### Example 2

### Construction of an insulin precursor by the polymerase chain reaction (PCR) with the codons more utilized by Pichia pastoris.

Primers:

SEQ ID: N° 7: 5´-ACTTGGTTGA AGCTTTGTAC TTGGTTTGTG

GTGAAAGAGG TTTCTTCTAC-3´

SEQ ID N° 8: 5´-AGAAGTACAA CATTGTTCAA CGATACCTCT

CTTAGTCTTT GGAGTGTAGA -3´

SEQ ID:N° 9: 5´-ACACTTGTGT GGTTCTCACT TGGTTGAAGC

TTT-3´

SEQ ID:N° 10: 5´- TTACTCGAGT TAGTTACAGT AGTTTTCCAA

TTGGTACAAA GAACAGATAG AAGTACAACA TTGTTC -3´

SEQ ID: N° 11: 5´-CCGCTCGAGA AGAGATTTGT TAACCAACAC

TTGTGT -3´

The obtained product contains the following sequence:

SEQ ID: N° 12:
5´-TTTGTTAACC AACACTTGTG TGGTTCTCAC TTGGTTGAAG
CTTTGTACTT GGTTTGTGGT GAAAGAGGTT TCTTCTACAC TCCAAAGACT
AAGAGAGGTA TCGTTGAACA ATGTTGTACT TCTATCTGTT CTTTGTACCA
ATTGGAAAAC TACTGTAACT AA-3´

The PCR conditions were identical to the ones of Example 1.
1- The twentieth part of the product obtained in PCR was employed as template for the subsequent event.
2- The final PCR product was purified by microspin S300 column (Amersham) and digested with the Xho I restriction enzyme.
   The digestion product was ligated to the pPIC9 vector that was previously digested with the restriction enzyme Xho I.
3- A digestion with the Hpa I restriction enzyme was carried out for detecting the recombinant clones and the correct orientation of the insert.

### Example 3

### Construction of Factor α with preferences codons of Pichia pastoris

By means of this technique the nucleotide sequence corresponding to the leader sequence or signal peptide was cloned.

The employed primers were the following:

SEQ ID: N°. 13: 5´-CGCGGATCCA AACCATGAGA TTCCCATCTA
TCTTCACTGC TGTTTTGTTC GCTGCT -3´

SEQ ID: N°. 14: 5´- GTTTTGTTCG CTGCTTCTTC
TGCTTTGGCT GCTCCTGTTA ACACTACTAC TGAAGACGAA ACTGCTCA-3´

SEQ ID: N°. 15: 5´-ACGTCGAAGT CACCTTCCAA GTCAGAGTAA
CCGATAACCG CTTCAGCTGG GATTTGAGCA GTTTCGTCTT C -3´

SEQ ID: N°. 16: 5´-GATGAACAAC AAACCATTAT TAGTAGAGTT
AGAGAAAGGC AAAACAGCAA CGTCGAAGTC ACCTTC -3´

SEQ ID: N°. 17: 5´-CCGCTCGAGA GAAACACCCT CTTCCTTAGC
AGCGATAGAA GCGATAGTAG TGTTGATGAA CAACAAACCA TT -3´

The final product has the following sequence:

SEQ ID N° 18

5´-ATGAGATTCC CATCTATCTT CACTGCTGTT TTGTTCGCTG

CTTCTTCTGC TTTGGCTGCT CCTGTTAACA CTACTACTGA AGACGAAACT

GCTCAAATCC CAGCTGAAGC GGTTATCGGT TACTCTGACT TGGAAGGTGA

CTTCGACGTT GCTGTTTTGC CTTTCTCTAA CTCTACTAAT AATGGTTTGT

TGTTCATCAA CACTACTATC GCTTCTATCG CTGCTAAGGA AGAGGGTGTT

TCTCTCGAGA AGAGAGAGGC TGAAGCA-3´

Cloning of the MFα and the insulin precursor with preferences codons of *Pichia pastoris:*
1- The signal peptide pPIC9 was replaced by a signal peptide with *Pichia pastoris* codons. pPIC9 was digested with restriction enzymes BamHI y XhoI
2- The digested fragments were separated in 0,8% agarose gel and the 7780 bp. fragment was recovered.
3- The PCR product SEQ ID: N° 18 was digested with the same restriction enzymes utilized in 1 and was ligated to the fragment obtained in 2.
4- The vector obtained in 3 and the PCR fragment SEQ ID N° 12 was digested with the XhoI, and subsequently they were ligated.
5- The recombinants having the correct orientation of the insulin precursor insert were detected by the HpaI.

### EXAMPLE 4

### Cloning of insulin precursor gene in pPIC9 yeasts vector

The DNA fragment encoding the insulin precursor was amplified by PCR, employing as a template SEQ N° 6 previously obtained and as primers the following sequences:

SEQ ID: N° 19. 5´ -GGGATCCAT ATGCTCGAGA AAAGATTTGT
GAACCAACAC CTGT-3´.

SEQ ID: N° 20. 5´ -TTAGAATTCC CGGGTCTAGT TGCAGTAGTT
CT- 3´.

The obtained PCR product was purified by employing the DNA clean up system Kit (Promega), according to the manufacturer instructions.

The JM-109 *E. coli* strain was transformed with vector pPIC9.

Subsequently, the plasmid DNA was removed by using the Wizard plus miniprep DNA purification system Kit (Promega).

The vector and the insert were digested with Xho I and Eco RI and both were ligated according to conventional protocols.

5 µl of ligation product were utilized for transforming 100 µl of competent bacteria corresponding to Jm-109 *E. coli*. strain according to conventional protocols.

The DNA was recovered from the colonies resistant to ampiciline by the above disclosed method.

200 ng of DNA of each sample were digested with 5 U of Alw NI restriction enzyme or with 5 U of Xho I and Eco RI enzymes.

The colonies containing the recombinant plasmids were grown and the plasmid DNA was recovered and purified.

Subsequently, the plasmid DNA was sequenced. The primers employed in the sequencing of strand 5' - 3' were the following: 5' AOX1 and α-Factor. The strand 3' - 5' was sequenced by means of the primer 3' AOX1 (sequences provided by the Kit from Invitrogen, called 3'AOX1, 5'AOX1 and α-Factor.

The DNA required for this sequence was purified by means of a miniprep SV Kit (Promega). Between 3 - 5 µg of DNA were employed per sequencing and the employed protocol was that one suggested by the Amersham Kit.

### Example 5:

### Cloning strategy for insulin precursor in pPICZαA yeasts vector.

In this example, the cloning of a copy of a gene encoding the human insulin precursor, in pPICZαA, is illustrated.

The selected vector is the pPICZαA the general map thereof being shown in figure 2. This vector has 2 sites XhoI, one of them being located in the multiple cloning site (1185) while the other one is located in position 1247. The vector was digested with XhoI and the gene of interest was cloned according to the following protocol:

| | |
|---|---|
| PPICZαA | 10 µl (≅ 2µg) |
| Buffer Neb2 (10x) | 4 µl |
| H₂O | 23,6 µl |
| BSA (100 X) | 0,4 µl |
| Xho I | 2 µl (40 U) |

The digestion was carried out at 37° C for 6 hours. 40 µl of digestion product were applied to a column of HR S-200 microspin (Amersham).

Subsequently, a dephosphatizing was carried out with intestinal alkaline phosphatase or CIP according to the following protocol:

| | |
|---|---|
| pPICZαA (digested) | 40 µl |
| buffer NebCIP (10x) | 5 µl |
| H₂O | 4 µl |
| CIP | 1 µl |

The reaction was carried out at 37° C for 30 minutes. Finally, the reaction was stopped by heat (75°C for 10 minutes) and the DNA was purified by applying the same to a column of microspin HR S-400.

### Insert preparation

The insulin precursor was amplified by PCR by utilizing the same conditions employed in example 4 corresponding to the cloning of this sequence in vector pPIC9 with the following primers:

SEQ ID: N° 19: 5´ - GGGGATCCAT ATGCTCGAGA
AAAGATTTGT GAACCAACAC CTGT-3´
SEQ ID: N° 21: 5´-TCACTCGAGC GGTCTAGTTG CAGTAGTTCT-
3´

50 µl of PCR product were purified by applying to a column of microspin HR S-200. The product was digested for 6 hours according to the following protocol:

| | |
|---|---|
| PCR products | 40µl (≅ 600 ng) |
| Buffer Neb2 (10 X) | 5 µl |
| H₂O | 3 µl |
| BSA (100 X) | 0,5 µl |
| Xho I | 1,5 µl (40 U) |

The digestion was stopped by heat (65° C, for 20 minutes) and the digestion products were purified by a column of microspin HR S-200.

The ligation of the insulin precursor fragment to the to the vector pPICZαA was carried out according with the following protocol:

The vector and the insert were digested with Xho I and were again quantified for carrying out the ligation. The ligation was carried out with 100 ng of vector in each event, by utilizing the following molar relations vector/insert 1:1, 1:3, 1:6 and 1:0 (negative control).

Bacteria *E. coli* of Top 10 strain (Invitrogen), were transformed with 5 µl of each of the above mentioned relations.

In the plate corresponding to relation 1:3, 13 colonies were obtained vs 6 colonies obtained in the negative control. 13 colonies were picked in tubes with 1,5 ml of LB medium for preparing conventional minipreps.

The obtained DNAs were digested with de AlwNI for determining the number and orientation of the obtained recombinants.

7 recombinant colonies were obtained, with two of them in the correct orientation, thus obtaining the vector pPICZαA Ib (Figure 2).

The DNA of one of the colonies was utilized for transforming the TOP10 strain.

### Example 6:

### Multimeric cloning strategy of human insulin precursor in a yeast vector.

This example discloses the obtaining of a multicassette containing multiple copies of the gene encoding the insulin precursor in vector pPICZαA Ib obtained in Example 5.

The process followed to obtain the construction with two copies of the gene of interest is that one disclosed, as a multimerics generating protocol *in vitro* according to the detailed instructions provided by the manufacturer (Invitrogen), as follows:

Two digestions were carried out:
Digestion 1: pPICZαA Ib with Bam HI
Digestion 2: pPICZαA Ib with Bam HI and Bgl II.

The expression cassette was recovered from an agarose gel.

The cassette Bgl II-BamHI containing a copy of the insulin precursor gene was ligated with the product from digestion 1 and the bacteria *E. Coli* Top 10 were transformed.

The plasmid DNA was removed and the presence of recombinants was analysed by restriction mapping.

The two types of configurations were differentiated by restriction mapping with Bgl II and Bam HI. Those configurations in direct tandem were chosen for continuing the process. By means of this process a vector called pPICZαA Ib2 was generated, the vectors having the ends Bgl II and Bam HI compatibles for the ligation. However, both sites are destroyed when ligated.

The protocol of generation of multimerics *in vitro* was again employed by replacing the vector pPICZαA Ib by the vector pPICZαA Ib2, thus obtaining the vector pPICZαA Ib4 (vector with 4 copies of the gene in direct tandem).

Finally, a vector pPICZαA Ib8 was generated from the prior protocol by replacing the vector pPICZαA Ib4 by the vector pPICZαA Ib4.

For obtain of the cassette BglII-BamHI 4 µl of the DNA were digested with both enzymes simultaneously, at 37°C, overnight. Then, a gel was run with 0,8% agarose (Promega), in such a way that the cassette was separated with the multimerics of the remaining vector. For purifying the DNA fragment of the agarose, the corresponding band of the gel was split and it was purified according to the protocol of Clean-up Kit from Promega.

The recombinant clones always were detected with the ALwNI enzyme.

### Example 7:

### Yeasts transformations

The chosen strain for the transformation was *Pichia pastoris* GS115 (*His4*) (Invitrogen).

The transformation process was carried out according to the protocols of the instruction manual (pichia expression Kit; version G161219, 250043) provided by Invitrogen.

### Spheroplasts transformation process:

100 ul of spheroplast preparation (disclosed by Invitrogen) were utilized for each transformation event, and 10 ug of DNA (pPIC9-Ib) were added to the preparation. This preparation was incubated for 10 minutes at room temperature. During the incubation, 1 ml of 1:1 PEG/CaT solution was added to the cells and DNA solution. This preparation was homogenised and incubated for 10 minutes at room temperature.
After a centrifugation step at 750 X g for 10 minutes, the cellular pellet was re-suspended in 150 ul SOS medium and was kept for 20 minutes at room temperature. Then, 850 ul of 1M sorbitol were added and the cells were plated in agarose.

Several volumes (100 - 300 ul) of spheroplasts transformed with 10 ml of RD molten agarose were mixed and poured over plates containing RDB medium. Each sample was carried out by duplicate.

The plates were incubated at 28 - 30°C for 4 - 6 days. Samples were taken and the cellular viability was determined by cultivating the yeasts cells in a RDHB medium containing histidine.

### Example 8

### Selection and isolation of recombinant yeasts

The transformations of *Pichia pastoris* GS115 yeasts strains with vector pPic9 digested with *Bgl* II promotes the recombination in the locus AOX I. The replacement of the structural alcohol oxidase (AOX 1) gene occurs with a frequency of 5-35% of the transformers His⁺.

By means of replication experiment in plates on a minimum medium containing dextrose (MD) and a minimum medium containing methanol (MM), transformers Mut⁺ and Mut^{s} can be distinguished.

Colonies His⁺ from the transformation of example 7 were selected according to the following protocol:

Each colony was picked with a sterile tip and was applied on a MM plate by making a mark or strake, and then over a MD plate.

In order to differentiate both phenotypes the corresponding controls to GS115/His⁺ Mut⁺ and GS115/His⁺ Mut^{s} (Invitrogen) were included.

The plates were incubated at 30° C for 48 - 72 hours. This method permitted to distinguish the clones Mut^{s} as well as those Mut^{r} that normally grows in plate MD and MM.

Each of the clones Mut^{s} and Mut^{r} selected by this method was purified and pure clones were isolated. The isolation was carried out by effecting strakes of each colony in a minimum medium without histidine.

### Example 9

### Re-transformation of yeasts clones obtained in Example 8

The re-transformation of clones was carried out by employing the electroporation transformation method according to the protocol suggested by Invitrogen. The DNA utilized in the transformation corresponds to 20 µg of plasmid pPICZαA Ib.

### Example 10

### Identification and isolation of colonies producing insulin precursor, retransformed as in example 9.

Once the retransformation was finalized the presence of clones producing the insulin precursor was revealed by means of an immunochemical method.

Aliquots of 50 to 600 ul of transformed cells were spread in plates containing YPDS agar medium with 100ug/ml Zeocine.

Once the colonies resistant to Zeocine were grown, the presence of the insulin precursor was detected according to the following scheme:

On each plate under analyses a nitro cellulose membrane was placed in such a way that the membrane was in contact with each of the colonies and it was also deposited in an inverted form unto the culturing plates containing BMMY/agarose medium.

The plates were incubated with the filters adhered for 24 hours at 30°C.

Then, the membranes were removed and subjected to washing series with a solution of PBS/0.05% to 0.1% Tween-20 for an hour, changing the medium regularly.

The nitrocellulose membranes were blocked with 5% skimmed milk in PBS/0.1% Tween-20 for 1 hour at 4°C.

Subsequently, the membranes were incubated with a policlonal-Guinea pig antibody anti-insulin; for one hour at room temperature, and then were washed with PBS/0.1% Tween-20 solution for 30 minutes.

Subsequently, the filters were incubated with an anti Guinea pig IgG polyclonal antibody conjugated with peroxidase for 1 hour at room temperature and the filters were washed with a PBS/0.1% Tween-20 solution for 30 minutes. Finally, the presence of peroxidase was revealed with 0.012% H₂O₂ , 0.08% DAB; 100mM Tris/CLH, pH 7.5.

The colonies that resulted positive were identified and isolated from the original plate.

Based on the comparison of the reaction intensities, the high producing clones were selected.

### Example 11:

### Expression of the recombinant clones

In order to determining the productivity of the selected colonies, growing and induction experiments with BMGY/BMMY medium were carried out. The first culturing medium contains glycerol that is the carbon source utilized for the microorganism for producing biomass. The second culture medium contains methanol that is the inductor of AOXI promoter.

The colonies were grown in Erlenmeyer flasks in a BMGY medium at 30°C until reaching OD₆₀₀ₙₘ: 6 - 20. Then, the cells were centrifuged for replacing the cultured medium by BMMY in a volume corresponding to the fifth part of the volume utilized in the growing phase. The culturing was continued for 120 hours as from the medium change at a temperature of 30°C with stirring. Each 24 hours 0.5% v/v 100% methanol was added and samples were taken to be evaluated by electrophoresis in 15% polyacrilamide, Tris/Tricine gel. Each sample was centrifuged for removing the cells, supernatant was treated with a sample buffer according to the protocols provided by (Laemmli, U.K. *Nature* 227:680-685; 1970).

From the polyacrilamide gels those clones capable of secreting a peptide with an electrophoretic movility coincident with that one for the insulin precursor having a PM of between 5.800 to 5.900, were chosen.

The chosen clones shown a very high protein expression. Subsequently, the molecular characterization of the producing clone genome was carried out.

### Example 12

### Molecular characterization of recombinant clones

The extraction of *Pichia pastoris* DNA was carried out according to the method suggested by the Invitrogen guide.

### Southern Blot Analysis

The Southern Blot analysis was carried out according to the standard protocol.

Briefly, a 871 bp fragment AOX probe that is a fragment of AOX1 promoter obtained from the digestion of vector pPICZαA with enzymes Bg1II and HindI was utilized; and it was also utilized the following:

His probe that is a fragment 1587 bp of HIS4 gene obtained by digestion of vector Ppic9 with the MscI and the Ins probe that is a fragment 227 bp obtained by PCR employing as a template the plasmid PPIC9IB and the primers corresponding to the sequences SEQ ID: 15 and 16.

The chromosomal DNA was digested with the BglII.

In the filters hybridized with AOX probe the band about 1600 bp corresponding to AOX1 endogen gene was observed, not only in the non transformed GS115 yeasts but also in the other Mut^{r} clones. However, this band did not appear in the Mut^{s} transformed clones. In all transforming clones a band of 5700 bp corresponding to the expression cassette of insulin precursor under promoter AOX1 and HIS4 gene was observed, coming from the transformation with the pPIC9-Ib digested with BglII, with distinct intensity depending from the number of incorporated copies. In some clones, other bands having distinct sizes were observed and these bands could correspond, for example, to the lost of sites BglII by exonucleases previous to the insertion in the chromosome. In the clones coming from the retransformation of clone C2,7 with PPICZαA-Ib (clone B1, 3.3) (linearized with SacI), in addition to band 5.7 kbp, a band of 3.8 kbp corresponding to the insertion of the insulin precursor cassette under control of AOX1 promoter, and the zeocine gene was also observed.

### Detailed analysis of each clone hybridized with AOX probe.

*Pichia pastoris* GS115: expected band 1.6 kbp
Clone 25: expected band 1.6 and 5.7 kbp
Clone C1,46: expected band 1.6 and 5.7 kbp plus 3 bands of 7.8; 7.3 and 4.8 kbp.
Clone C2,7: expected band 5.7 kbp. Absent band of 1.6 kbp, indicating that this is a Mut^{s} transformer.
Clone B1,3.3: band of 5.7 kbp (insertion of C2,7) plus band of 3.8 kbp corresponding to insertion of pPICZaAIb cassette (linearized with SacI).

The same filters utilized with AOX probe were re-hybridized with the HIS probe. A band of 2.7 kbp corresponding to HIS4 endogenous gene was observed, not only in the non transformed yeasts GS115 but also in most of the transforming clones. This band did not appear in clone C1, 46 indicating in this case that there was an integration at the level of this gene thus loosing the sites BglII. In all the transforming clones a band of 5700bp was observed corresponding to the insulin precursor cassette under AOX1 promoter and HIS4 gene coming from the transformation with pPIC9-Ib digested with BglII, with different intensity depending on the number of incorporated copies. In some clones, other bands having different sizes were observed, this bands corresponding for example to the lost of sizes BglII by exonuclease digests previous to the insertion in the chromosome.

By the analysis of the Southern Blots hybridized with the HIS probe, a clone with only one copy of the expression cassette was individualized which clone was taken as a pattern for further determination of the number of copies in the other clones.

By the hybridization of the membranes with the insulin probe it was confirmed that all the bands obtained by hybridization with AOX and HIS probe excepting those corresponding to the endogenous genes, contained the gene corresponding to the insulin precursor.

### Dot Blot Analysis

For determining the number of copies of the sequence of the insulin precursor of the several transforming clones, the Dot Blot technique was employed, using Ins and Gap probes. The Gap probe was employed as a single copy gene pattern in all the clones. From the relationship between the signals obtained with both probes and taking as a reference an insulin single copy clone (obtained by analysis of Southern Blot), the number of copies of insulin was determined.

The number of copies of the gene encoding the insulin precursor in each clone was the following:
Clone 25: 6
Clone C1,46: 6
Clone C2,7: 1
Clone B1,3.3: 13
Clone V8,10.1: 8

### Characterization of Mut^{s} or Mut^{r} colonies by PCR:

A protocol according to the following scheme was utilized:

| | |
|---|---|
| Chromosomal DNA: | 10-20mg |
| 5'AOX | 0,5 µM |
| 3'AOX IN | 0,5 µM |
| dNTP | 0,2 mM |
| C12Mg | 1,5 mM |
| Taq: | 2U |
| Buffer 10X | 1x |

Sequence of primers:
5´AOX I: 5´ - GACTGGTTCC AATTGACAAG C (provided by
Invitrogen)
SEQ ID: N° 22 (3´ AOX IN): 5´ - GTCGTGGTTT
CTCATAGTAG AGTGGACA

The reaction conditions were the following: Denaturalization 94°C 2 minutes. 1 cycle
25 cycles
Denaturalization 94°C, 1 minute.
Annealing 55, 1 minute.
Extension 72°C, 1 minute.
Final extension: 72°C, 7 minutes. 1 cycle

The band of 730 bp appears in Mut^{r} clones. No band is observed in Mut^{s} clones.

### Quantification of the number of copies of the insulin precursor gene by PCR in the recombinant colonies.

DNA was extracted from all the samples and the quantity was normalized by means of PCR with Gap primers (gliceraldehyde, 3-phosfate dehidrogenase, single copy gene).

A new PCR was carried out with primers specific for insulin according to the prior quantification, by utilizing the analyzing increasing concentrations for each point. In this way, the signal saturation was avoided.

The PCR product was analyzed in a gel of 2% agarose and after staining with a etidio bromide the bands were visualized with an equipment for taking images fotodine. The quantification has been carried out with ImageQuant software.

As a unit the clone C2,7 was chosen, the unit having a single copy of the insulin precursor gene while the remaining clones were compared according with the intensity of the PCR products.

For guarantying that the experimental conditions of the amplification of the Gap genes and insulin was equivalent, primers have been designed having similar hybridization T° and similar sizes.

Gap primers:
SEQ ID: N° 23 (Gap5´): 5´ GGTCATCACT GCTCCATC
SEQ ID: N° 24 (Gap3´): 5´ AGCAGCACCA GTGGAAGAT

### PCR conditions:

Denaturalization: 94°C, 3 minutes
   24 cycles of:
   94°C, 1 minute
   56°C, 1 minute
   72°C, 30 seconds
Chromosomal DNA: 0,5 - 1,5 NG
Gap primers 5': 0,5 µM
   Gap 3': 0,5 µM
dNTP: 0,2 mM
ClMg: 1,5 mM
Taq Pol: 2,5 U
Buffer 10x: 1 x

The conditions for the insulin precursor were the same to the above described with the specific primers.

Insulin precursor primers:
SEQ ID: N° 19: 5´- GGGGATCCAT ATGCTCGAGA AAAGATTTGT
GAACCAACAC CTGT

SEQ ID: N° 21: 5´- TCACTCGAGC GGTCTAGTTG CAGTAGTTCT

The results from the experiments by Dot Blot and quantitative PCR were equivalent, in other words, the same number of copies of obtained recombinants was found for both methodologies.

### Example 13: Fermentation process

The fermentation was carried out not only in a fermentor BioFlo 3000 (New Brinswick Scientific) but also in a Biostat II (B.Braun Biotech). Both fermentors are provided with 2,5 liters glasses. However, the fermentation process may be adapted to higher volumes protocols.

### Culture preparation

The pre-culture for inoculating the fermentor was carried out in Erlenmeyer 125 ml flasks with 25 ml of BMGY culture medium, the same was inoculated with the corresponding strain, from frozen samples in 50% glycerol at - 80°C.

The culture was incubated at 30°C, 240 r.p.m. for 14 hours in an orbital stirrer.

### Fermentation

The total of 25 ml was transferred to the fermentor containing 1.2 L of BSM basal medium plus 4.35 ml/l of trace salts and 1% glycerol. The temperature was controlled to 30°C, the oxygen was dissolved at 35%, the pH was 4.5 and the aeration was 1 vvm. The dissolved oxygen was controlled by means of the variation according to PID control of the stirring velocity and addition of O₂. The pH was controlled by means of automatic addition of 28% ammonium hydroxide solution.

After approximately 16 hours of culturing, or when the optic density reached the value close to 20 unites of absorbency at 600 nm, the lots culturing phase was finalized.

The lot phase was begun by feeding the fermentor by means of the addition of 50% glycerol plus 12 ml/l of trace salts. The velocity of addition was regulated at 24 ml/l/h. This phase lasted approximately 20 hours, reaching values of OD: 300.

Once the growing phase of the biomass was finalized, the cells remained without feeding for half hour and the production phase was begun. During said phase the pH was regulated between 3.5 and 5.5, and 100% methanol was feed plus 12 ml/l of trace salts, at the velocity of 1.2 ml/l/h. This last phase can be extended up to 96 hours. Variations in the choosing of the adequate time for adding methanol to the culture, variations in the methanol concentration and variations in the inclusion of the double feeding glycerol/methanol, may be carried out for further improving the production process.

Once this step was finalized, the step of separation the cells from culture was begun. When the fermentation process was carried out with high volumes, appropriate separation methods were utilized.

The implementation of this fermentation protocol in 1 and 100. liters permitted to obtain between 200 and 400 mg of insulin precursor per liter of fermentation according to the quantity of methanol employed in the induction.

The supernatant was apt for being introduced in the first step of purification of the insulin precursor.

### Example 14:

### Purification of recombinant Human Insulin Capturing the precursor

The recovering of the recombinant human insulin precursor from the culture medium was carried out by means of cationic interchange chromatography, for example, SP Sepharose Fast Flow (Pharmacia) (Katsoyannis, P. G., y col. *Biochemistry* 6:2642-2655; 1967) or by means of other adsorptive chromatographic technique, such us for example the Hydrophobic Interaction Chromatography by utilizing a Phenyl Sepharose Fast Flow resin, according to the protocols disclosed by Gagnon, Pete et Al. Large Scale Process Development for Hydrophobic Interaction Chromatography, Part 1: gel Selection and Development of Binding Conditions. BioPharm 8:21-29; 1995.

The washing Buffer consisted of a solution of 50 mM sodium acetate and 50 mM NaCl, and the elution buffer consisted of 50 mM sodium acetate and 450 mM NaCl. The precursor was maintained soluble by the addition of ethanol or urea.

During the process, the column was equilibrated by 3 Vc of washing buffer as a lineal velocity of 100 cm/h. The linked of the product was carried out at a lineal velocity of 90 to 120 cm/h. Once this step was concluded, a washing with 4 Vc of washing buffer was carried out. The product was eluted with 10 Vc of eluting buffer. The chromatographic process was monitored by OD at 280 nm. Those fractions containing the product of interest were collected in a single solution.

### Enzymatic processing of the insulin precursor Digestion with trypsine and carboxipeptidase B

The digestion was carried out by adjusting the concentration of the precursor solution between 1 and 20 mg/ml, according to what is disclosed in the European Patent N° 195691. The reaction prosecution was monitored by means of. RP-HPLC. The digestion was stop with 7.5 M acetic acid.

Proteases were eliminated from the reaction medium by molecular exclusion (chromatography or ionic interchange chromatography at pH: 2-5). The fractions corresponding to the.digested precursor were collected in a single solution for its further digestion with carboxipeptidase B according the European Patent 195691.

As an alternative method, the simultaneous addition of both enzymes was carried out by following the protocols disclosed in the European Patent 195691 and in the publication Lila R. Castellanos-Serra et Al. *FEBS Letters* 378: 171-176; 1996.

The final purification of the insulin obtained after the enzymatic action can be carried out by any chromatographic technique such as the ones disclosed in the US Patent N° 5.663.291; EPO N° 195691; and the techniques disclosed in the publication Kroeff; Eugene et Al. Journal of Chromatography. 461:45-61: 1989.

While preferred embodiments of the present invention have been illustrated and described, it will be obvious to those skilled in the art that various changes and modifications may be made therein without departing from the scope of the invention as defined in the appended claims.

## Claims

1. Methylotrophic recombinant yeast strain for producing human insulin precursor, the strain having a genome comprising a copy of a first DNA construction and a second DNA construction, wherein said constructions controlling the expression and secretion of human insulin precursor, said DNA constructions comprising at least one DNA sequence encoding a human insulin precursor and analogous thereof.

2. The yeast strain of claim 1, wherein the strain secreted a human insulin precursor of the formula:
B(1-30)-Y1-Y2-A(1-21), wherein Y1 is lysine or arginine; Y2 is lysine or arginine; B(1-30) is the B peptide of the human insulin; and B(1-21) is the A peptide of human insulin.

3. The yeast strain of claim 1, wherein the strain is a member selected from *Hansenula, Pichia, Candida,* and *Torulopsis.*

4. The yeast strain of claim 3, wherein the strain is yeast *Pichia Pastoris* deposited under number ATCC PTA-2260.

5. The yeast strain of claim 1, wherein the first DNA construction comprises:
a1) a first insertable DNA sequence corresponding to a 5' regulatory region (promoter) operably linked to
b1) an exporting signal sequence operable linked to
c1) a sequence encoding a human insulin precursor operable linked to
d1) a 3' termination sequence linked to
e1) a selectable gene linked to
f1) a second insertable DNA sequence; and
the second DNA construction comprises:
a2) a first insertable DNA sequence corresponding to a 5' regulatory region (promoter) operably linked to
b2) an exporting signal sequence operable linked to
c2) a sequence encoding a human insulin precursor operable linked to
d2) a 3' termination sequence linked to
e2) a selectable gene distinct from the selectable gene of the first DNA construction.

6. The first DNA construction of claim 5, comprising at least one expression cassette for expressing the human insulin precursor, the cassette comprising:
a) a 5' regulatory region operably linked to
b) a DNA sequence encoding a signal sequence operable linked to
c) a sequence encoding a human insulin precursor operable linked to
d) a functional termination sequence.

7. The first DNA construction of claim 5, wherein the selectable gene is HIS4.

8. The first DNA construction of claim 5, wherein 5' and 3' ends of said DNA construction comprises sequences enough homologous with a target gene of the yeast to permit the replacement by a specific insertion of the DNA construction in the target gene, in the same relative orientation of the target gene in the yeast genome.

9. The first DNA construction of claim 5, wherein the 5' regulatory region is selected from the group consisting of the *Pichia pastoris* primary alcohol oxidase enzyme (AOX1) gene promoter, the secondary alcohol oxidase II enzyme (AOX2) gene promoter, the *Pichia pastoris* dihydroxyacetone synthase (DAS) gene promoter, the *Pichia pastoris* p40 regulatory regions promoter, the *Pichia pastoris* catalase promoter, and the glyceraldehyde dehydrogenase GAP promoter.

10. The first DNA construction of claim 5, wherein the signal sequence is the MF α of *Sacharomyces cerevisiae.*

11. The first DNA construction of claim 5, wherein the functional termination sequence is the termination sequence derived from *Pichia pastoris* AOX1 gene.

12. The first DNA construction of claim 5, further comprising:
a) a first insertable DNA sequence corresponding to a 5' regulatory region of *Pichia pastoris* AOX1 gene operably linked to
b) the MF α signal sequence of *Sacharomyces cerevisiae* operable linked to
c) the sequence encoding the human insulin precursor of formula B(1-30)-Y1-Y2-A(1-21) operable linked to
d) a 3' termination sequence of *Pichia pastoris* AOX1 gene operably linked to
e) a *Pichia pastoris* HIS4 selection gene operably linked to
f) a second insertable DNA sequence corresponding to *Pichia pastoris* AOX1 gene termination sequence.

13. The first DNA construction of claim 5, wherein the sequence encoding the human insulin precursor is cloned in said construction following the protease site of the signal peptide, wherein all the secreted human insulin precursor contains, in its amino terminal end, the first amino acid of the human insulin precursor.

14. The first DNA construction of claims 5-13, wherein said DNA construction is incorporated into a vector selected from the group consisting of linear and circular vectors.

15. The second DNA construction of claim 5, comprising at least one expression cassette for expressing the human insulin precursor, the cassette comprising:
a) a 5' regulatory region operably linked to
b) a DNA sequence encoding a signal sequence operable linked to
c) a sequence encoding a human insulin precursor operable linked to
d) a functional termination sequence.

16. The second DNA construction of claim 5, further comprising a selectable gene distinct from the selectable gene of the first DNA construction, wherein said selectable gene is the gene encoding zeocine resistance and wherein said selectable gene permits to carry out a second selection event.

17. The second DNA construction of claim 5, wherein 5' end of said DNA construction comprises a single sequence enough homologous with a target gene of the yeast to permit the integration of the DNA construction in the target gene, in a single event.

18. The second DNA construction of claim 5, wherein the 5' regulatory region is selected from the group consisting of the *Pichia pastoris* primary alcohol oxidase enzyme (AOX1) gene promoter, the secondary alcohol oxidase II enzyme (AOX2) gene promoter, the *Pichia pastoris* dihydroxyacetone synthase (DAS) gene promoter, the *Pichia pastoris* p40 regulatory regions promoter, the *Pichia pastoris* catalase promoter, and the glyceraldehyde dehydrogenate GAP promoter.

19. The second DNA construction of claim 5, wherein the signal sequence is MF α *Sacharomyces cerevisiae.*

20. The second DNA construction of claim 5, wherein the functional termination sequence is the termination sequence derived from *Pichia pastoris* AOX1 gene.

21. The second DNA construction of claim 5, further comprising:
a) a first insertable DNA sequence corresponding to a 5' regulatory region of *Pichia pastoris* AOX1 gene operably linked to
b) the MF α signal sequence of *Sacharomyces cerevisiae* operable linked to
c) the sequence encoding the human insulin precursor of formula B(1-30)-Y1-Y2-A(1-21) operable linked to
d) a 3' termination sequence of *Pichia pastoris* AOX1 gene operably linked to
e) the zeocine-resistant selection gene.

22. The second DNA construction of claim 5, wherein the sequence encoding the human insulin precursor is cloned in said construction following the protease site, wherein all the secreted human insulin precursor contains, in its amino terminal end, the first amino acid of the human insulin precursor.

23. The second DNA construction of claims 15-22, wherein said DNA construction is incorporated into a vector selected from the group consisting of linear and circular vectors.

24. A method of obtaining the yeast strain of claim 1, comprising the steps of:
i) transforming a yeast cell with a first DNA construction comprising:
a) a first insertable DNA sequence corresponding to a 5' regulatory region of *Pichia pastoris* AOX1 gene operably linked to
b) the MF α signal sequence of *Sacharomyces cerevisiae* operable linked to
c) the sequence encoding the human insulin precursor of formula B(1-30)-Y1-Y2-A(1-21) operable linked to
d) a 3' transcription termination sequence of *Pichia pastoris* AOX1 gene operably linked to
e) a *Pichia pastoris* HIS4 selection gene operably linked to
f) a second insertable DNA sequence corresponding to *Pichia pastoris* AOX1 gene termination sequence;
ii) selecting the yeast cells;
iii) isolating a yeast strain;
iv) re-transforming the yeast strain obtained in steps i)-iii) with a second DNA construction comprising:
a) a first insertable DNA sequence corresponding to a 5' regulatory region of *Pichia pastoris* AOX1 gene operably linked to
b) the MF *α* signal sequence of *Sacharomyces cerevisiae* operably linked to
c) the sequence encoding the human insulin precursor of formula B(1-30)-Y1-Y2-A(1-21) operable linked to
d) a 3' termination sequence of *Pichia pastoris* AOX1 gene operably linked to
e) the zeocine-resistant selection gene;
v) selecting the re-transformed yeast strain; and
vi) isolating the selected and re-transformed yeast strain.


SEQUENCE LISTING

<110> Laboratorios BETA S.A.

<120> Expression of a human insulin precursor in P. pastoris

<130> human insulin precursor

<140>
<141>

<150> AR- P000104797
<151> 2000-09-13

<160> 24

<170> PatentIn Ver. 2.0

<210> 1
<211> 36
<212> DNA
<213> Artificial Sequence

<220>
<223> Description of Artificial Sequence :PCR primer

<220>
<223> Description of Artificial Sequence: PCR primer

<400> 1

tcacacctgg tggaagctct ctacctagtg tgcggg                                36

<210> 2

<211> 45

<212> DNA

<213> Artificial Sequence

<220>

<223> Description of Artificial Sequence: PCR primer

<400> 2

ggtcttgggt gtgtagaaga agcctcgttc cccgcacact aggta                      45

<210> 3

<211> 39

<212> DNA

<213> Artificial Sequence

<220>

<223> Description of Artificial Sequence: PCR primer

<400> 3

tttgtgaacc aacacctgtg cggctcacac ctggtggaa                             39

<210> 4

<211> 45

<212> DNA

<213> Artificial Sequence

<220>

<223> Description of Artificial Sequence: PCR primer

<400> 4

gctggtacag cattgttcca caatgccacg cttggtcttg ggtgt                    45

<210> 5

<211> 52

<212> DNA

<213> Artificial Sequence

<220>

<223> Description of Artificial Sequence: PCR primer

<400> 5

ctagttgcag tagttctcca gctggtagag ggagcagatg ctggtacagc at            52

<210> 6

<211> 162

<212> DNA

<213> Artificial Sequence

<220>

<223> Description of Artificial Sequence: PCR product
      corresponding to the complete insulin precursor

<400> 6

tttgtgaacc aacacctgtg cggctcacac ctggtggaag ctctctacct agtgtgcggg 60

gaacgaggct tcttctacac acccaagacc aagcgtggca ttgtggaaca atgctgtacc 120

agcatctgct ccctctacca gctggagaac tactgcaact ag 162

<210> 7

<211> 50

<212> DNA

<213> Artificial Sequence

<220>

<223> Description of Artificial Sequence: PCR primer

<400> 7

acttggttga agctttgtac ttggtttgtg gtgaaagagg tttcttctac 50

<210> 8

<211> 50

<212> DNA

<213> Artificial Sequence

<220>

<223> Description of Artificial Sequence: PCR primer

<400> 8

agaagtacaa cattgttcaa cgatacctct cttagtcttt ggagtgtaga 50

<210> 9

<211> 33

<212> DNA

<213> Artificial Sequence

<220>

<223> Description of Artificial Sequence: PCR primer

<400> 9

acacttgtgt ggttctcact tggttgaagc ttt                                33

<210> 10

<211> 66

<212> DNA

<213> Artificial Sequence

<220>

<223> Description of Artificial Sequence: PCR primer

<400> 10

ttactcgagt tagttacagt agttttccaa ttggtacaaa gaacagatag aagtacaaca 60

ttgttc                                                              66

<210> 11

<211> 36

<212> DNA

<213> Artificial Sequence

<220>

<223> Description of Artificial Sequence: PCR primer

<400> 11

ccgctcgaga agagatttgt taaccaacac ttgtgt                             36

<210> 12

<211> 162

<212> DNA

<213> Artificial Sequence

<220>

<223> Description of Artificial Sequence: sequence
      corresponding to the complete insulin precursor
      modified according to the P. pastoris codon usage

<400> 12

tttgttaacc aacacttgtg tggttctcac ttggttgaag ctttgtactt ggtttgtggt 60

gaaagaggtt tcttctacac tccaaagact aagagaggta tcgttgaaca atgttgtact 120

tctatctgtt ctttgtacca attggaaaac tactgtaact aa                    162

<210> 13

<211> 56

<212> DNA

<213> Artificial Sequence

<220>

<223> Description of Artificial Sequence:oligonucleotide

<400> 13

cgcggatcca aaccatgaga ttcccatcta tcttcactgc tgttttgttc gctgct     56

<210> 14

<211> 68

<212> DNA

<213> Artificial Sequence

<220>

<223> Description of Artificial Sequence:oligonucleotide

<400> 14

gttttgttcg ctgcttcttc tgctttggct gctcctgtta acactactac tgaagacgaa 60

actgctca 68

<210> 15

<211> 71

<212> DNA

<213> Artificial Sequence

<220>

<223> Description of Artificial Sequence:oligonucleotide

<400> 15

acgtcgaagt caccttccaa gtcagagtaa ccgataaccg cttcagctgg gatttgagca 60

gtttcgtctt c 71

<210> 16

<211> 66

<212> DNA

<213> Artificial Sequence

<220>

<223> Description of Artificial Sequence:oligonucleotide

<400> 16

gatgaacaac aaaccattat tagtagagtt agagaaaggc aaaacagcaa cgtcgaagtc 60

accttc 66

<210> 17

<211> 72

<212> DNA

<213> Artificial Sequence

<220>

<223> Description of Artificial Sequence:oligonucleotide

<400> 17

ccgctcgaga gaaacaccct cttccttagc agcgatagaa gcgatagtag tgttgatgaa 60

caacaaacca tt 72

<210> 18

<211> 267

<212> DNA

<213> Artificial Sequence

<220>

<223> Description of Artificial Sequence: MF alpha from

S. cerevisiae modified according to the P.

pastoris codon usage

<400> 18

atgagattcc catctatctt cactgctgtt ttgttcgctg cttcttctgc tttggctgct 60

cctgttaaca ctactactga agacgaaact gctcaaatcc cagctgaagc ggttatcggt 120

tactctgact tggaaggtga cttcgacgtt gctgttttgc ctttctctaa ctctactaat 180

aatggtttgt tgttcatcaa cactactatc gcttctatcg ctgctaagga agagggtgtt 240

tctctcgaga agagagaggc tgaagca 267

<210> 19

<211> 44

<212> DNA

<213> Artificial Sequence

<220>

<223> Description of Artificial Sequence: PCR primer

<400> 19

ggggatccat atgctcgaga aaagatttgt gaaccaacac ctgt 44

<210> 20

<211> 32

<212> DNA

<213> Artificial Sequence

<220>

<223> Description of Artificial Sequence: PCR primer

<400> 20

ttagaattcc cgggtctagt tgcagtagtt ct 32

<210> 21

<211> 30

<212> DNA

<213> Artificial Sequence

<220>

<223> Description of Artificial Sequence: PCR primer

<400> 21

tcactcgagc ggtctagttg cagtagttct 30

<210> 22

<211> 28

<212> DNA

<213> Artificial Sequence

<220>

<223> Description of Artificial Sequence: PCR primer

<400> 22

gtcgtggttt ctcatagtag agtggaca 28

<210> 23

<211> 18

<212> DNA

<213> Artificial Sequence

<220>

<223> Description of Artificial Sequence: PCR primer

<400> 23

ggtcatcact gctccatc 18

<210> 24

<211> 19

<212> DNA

<213> Artificial Sequence

<220>

<223> Description of Artificial Sequence: PCR primer

<400> 24

agcagcacca gtggaagat 19